# EUROPEAN PATENT SPECIFICATION

(11) **EP 1 086 149 B1**
(45) Date of publication and mention of the grant of the patent: **29.07.2009**
(21) Application number: 99921514.8
(22) Date of filing: 29.04.1999
(51) Int. Cl.: C08F 8/00, A61F 2/00, B32B 27/00

(54) **SURFACTANTS THAT MIMIC THE GLYCOCALYX**
OBERFLÄCHENAKTIVE STOFFE DIE GLYCOCALYX IMITIEREN
TENSIOACTIFS PRESENTANT UN MIMETISME PAR RAPPORT AU GLYCOCALYX

(30) Priority: 29.04.1998 US 83544 P
(43) Date of publication of application: 28.03.2001
(73) Proprietor: Marchant, Roger E., Cleveland Heights, OH 44106 (US); Zhang, Tianhong, Rockport, ME 04856 (US); Qiu, Yongxing, Atlanta, GA 30338 (US); Ruegsegger, Mark A., Cleveland Heights, OH 44121 (US)
(72) Inventor: Marchant, Roger E., Cleveland Heights, OH 44106 (US); Zhang, Tianhong, Rockport, ME 04856 (US); Qiu, Yongxing, Atlanta, GA 30338 (US); Ruegsegger, Mark A., Cleveland Heights, OH 44121 (US)
(74) Representative: Stuart, Ian Alexander
(86) International application number: PCT/US1999/009258
(87) International publication number: WO 1999/055742

(56) References cited:
- US-A- 5 897 955
- N.B. HOLLAND, Y. QIU, M. RUEGSEGGER, R.E. MARCHANT: "Biomimetic engineering of non-adhesive glycocalyx-like surfaces using oligosaccharide surfactant polymers" NATURE, vol. 392, no. 23, 23 April 1998 (1998-04-23), pages 799-801, XP002195981
- Y. QIU, T. ZHANG, M. RUEGSEGGER, R.E. MARCHANT: "Novel nonionic oligosaccharide surfactant polymers derived from poly(vinylamine with pendant dextran and hexanoyl groups)" MACROMOLECULES, vol. 31, no. 1, 13 January 1998 (1998-01-13), pages 165-171, XP002195982

## Description

The present invention provides comblike surfactant polymers that are useful for changing the surface properties of synthetic biomaterials, particularly implantable biomaterials.

The use of synthetic biomaterials to sustain, augment, or completely replace diseased human organs has increased tremendously over the past thirty years. Synthetic biomaterials are used in synthetic implants such as vascular grafts, heart valves, and ventricular assist devices that have cardiovascular applications. Synthetic biomaterials are also used in extracorporeal systems and a wide range of invasive treatment and diagnostic systems. Unfortunately, existing biomaterials suffer from well-known problems associated with surface-induced thrombosis or clot formation, such as thrombotic occlusion and thromboemboli, and infection.

There have been several attempts to create nonthrombogenic surfaces on synthetic implants thereby increasing the blood-biocompatibility of implants. Early attempts included precoating the implants with proteins not involved in thrombosis, such as albumin, to mask the thrombogenic surface of the implant. However, such implants lose their nonthrombogenic properties within a short time. Attempts have been made to mask the thrombogenic surface by coating gelatin onto implants such as ventricular assist devices. While the gelatin coating reduced the thrombus formation, it did not adhere to the implant and it did not prevent thromboemboli and infection.

Attempts have been made to render implants nonthrombogenic by coating the surface of the implant with polyethylene oxide to mask the thrombogenic surface of the implant. At times this treatment reduced protein adsorption and thrombogenesis. However, the coupling of polyethylene oxide to the surface of the implant involves complex chemical immobilization procedures. Moreover, the coated implants do not consistently exhibit protein resistance because of the lack of control over the density of immobilized polyethylene oxide.

There have been many attempts to prepare nonthrombogenic surfaces by attaching heparin to biomaterials. However, each method requires complex immobilization procedures such that the implant surface be first modified by attachment of a coupling molecule before heparin can be attached. For example, the positively charged coupling agent tridodecylmethylammonium chloride (TDMAC) is coated onto an implant, which provides a positively charged surface and allows heparin which has a high negative charge density, to be attached. However, the heparin slowly dissociates from the surface, to expose the positively charged TDMAC surface, which is particularly thrombogenic. Thus, the TDMAC heparin coated implant is successful only for short term implants such as catheters.

Despite these considerable research efforts, synthetic biomaterials and medical devices made from such biomaterials still suffer well-known problems associated with surface-induced thrombosis and infection. Accordingly, it is desirable to have new materials that can be used to coat biomaterials and to change their surface properties. Materials that are useful for preventing undesirable adhesions, such as proteins, or promoting desirable adhesions, such as endothelial cells are especially desirable.

### SUMMARY OF THE INVENTION

In accordance with the present invention, novel, comblike, surfactant polymers which are useful for changing the surface properties of biomaterials are provided. Such surfactant polymers comprise a polymeric backbone of repeating monomeric units coupled to side chains which are :(a) a plurality of hydrophobic side chains and (b) a plurality of hydrophilic side chains. The hydrophobic side chains comprise an alkyl group (CH₃(CH₂-)ₙ) comprising from about 2 to 18 methylene groups. The alkyl groups are linked to the polymeric backbone through one or more of ester linkages, secondary amine linkages, or, preferably, amide linkages. The hydrophilic side chain comprises an oligopeptide of from about 3 to about 30 amino acid residues, said oligopeptide having an amino acid sequence which interacts with protein receptors on the surface of cells. There may be a second plurality of hydrophilic side chains selected from neutral oligosaccharides, which, preferably, have weight average molecular weight of less than 7000; and charged oligosaccharides, preferably negatively charged having a weight average molecular weight of less than 10,000. A preferred amino acid sequence which is known to interact with molecules on the surface of cells is arginine (R), glycine (G), and aspartic acid (D). The hydrophilic side chains are linked to the polymeric backbone through one or more of ester linkages, secondary amine linkages, or, preferably, amide linkages. It has been shown that effective surface modification of biomaterials can be accomplished using the surfactant polymers of the present invention without any requirements for surface coupling chemistries.

The present invention also provides a method of promoting adhesion of animal cells to a surface of an implantable, biomaterial by attaching a comblike surfactant polymer of the present invention to a hydrophobic surface thereof.

### BRIEF DESCRIPTION OF THE FIGURES

- Figure 1: is a schematic representation of the synthetic route to poly(vinyl amine) and the derivatization of poly(vinyl amine) with (1) dextran lactone and (2) (N-hexanoyloxy)succinimide.
- Figure 2: shows phase contrast images showing the attachment and growth of human umbilical vein endothelial cells on surfactant coated surfaces after 72 hours.

### DETAILED DESCRIPTION OF THE INVENTION

The present invention provides novel comblike surfactant polymers that mimic the glycocalyx. The glycocalyx is the oligosaccharide-rich region on the surface of cells. The glycocalyx serves to prevent undesirable biological adhesions, while proteins embedded in the cell membrane glycocalyx serve to promote desirable specific adhesions.

The comblike surfactant polymers of the present invention comprise a (preferably flexible) polymeric backbone which is linked to a plurality of hydrophobic side chains and to a plurality of hydrophilic side chains. The polymeric backbone is preferably conformationally flexible. Preferably, the polymeric backbone is formed from a homopolymer that contains a plurality of functional side groups such as for example OH groups, COOH groups, or NH₂ groups. Although less preferred, the polymeric backbone may be formed from a copolymer which has a combination of functional side groups. For example, the copolymer may have OH side groups and NH₂ side groups. Suitable homopolymers for forming the comblike surfactant polymer are, by way of example, polylysine, poly(vinyl alcohol) or poly (vinyl amine). Preferably, the polymeric backbone is formed from a poly (vinyl amine).

The hydrophobic side chains comprise from about 2 to 18 methylene groups, preferably from about 4 to about 12 methylene groups, more preferably from about 4 to about 10 methylene groups, and are linked to the polymeric backbone via an ester linkage, a secondary amine linkage, or, preferably, an amide linkage. Preferably, the hydrophobic side chains are attached to the polymeric backbone by reacting an alkanoyl (CH₃(-CH₂-)ₙCO-)or an alkanal (CH₃(CH₂-)ₙCHO)with the homopolymer of the backbone using conventional procedures. For example, a plurality of hydrophobic side chains may be linked to poly(vinyl amine) by standard procedures that use the corresponding N-alkanoyloxy succinimide and poly(vinyl amine) as reactants.

The hydrophilic side chains comprises an oligopeptide of from about 3 to about 30 amino acid residues, said oligopeptide having an amino acid sequence which interacts with protein receptors on the surface of cells; and combinations thereof. The hydrophilic side chains are linked to the polymeric backbone through an ester linkage, a secondary amine linkage, or preferably an amide linkage. They may also include a neutral oligosaccharide which preferably has a weight average molecular weight of less than 7000; and/or a charged oligosaccharide, preferably a negatively charged oligosaccharide having a weight average molecular weight of less than 10,000. In a preferred embodiment a plurality of oligopeptides and a plurality of oligosaccharides are attached to the polymeric backbone.

To form a coating which blocks adhesion of non-specific plasma proteins on the surface of the substrate, the surfactant polymer, preferably includes a plurality of hydrophilic side chains formed from oligosaccharides. Such surfactant polymers may be non-ionic or ionic. Suitable oligosaccharides include, but are not limited to, neutral oligosaccharides having a weight average molecular weight of less than about 7000. Examples of such neutral oligosaccharides include dextran, which is composed of α(1→6) linked glucose residues, and an oligomaltose, which is composed of a (1→4) linked glucose residues. Such oligosaccharide side chains are attached to the reactive amines of the poly(vinyl amine) by standard procedures that employ the polymer and the corresponding lactone form of the oligosaccharide as reactants.

The hydrophilic side chains may also include ones formed from charged oligosaccharides such as, for example, the oligosaccharides that are obtained from heparin. The heparin oligosaccharides are hydrated and negatively charged which provides an additional electrostatic repulsive force that further repels plasma proteins and cellular elements such as platelets. The heparin oligosaccharide contains the unique pentasaccharide sequence that is essential for heparin's anticoagulant activity. The heparin product of deaminative cleavage of heparin possesses a terminal 2,5 anhydromanose unit. In a preferred embodiment, the terminal aldehyde of the 2,5 anhydromannose is reacted with the amines on the polymeric backbone via reductive amination to form a secondary amine.

Other suitable charged oligosaccharides for forming coatings which are non-adhesive for plasma proteins include dermatan sulfate, and dextran sulfate, which are hydrated and negatively charged and serve to repulse proteins and platelets. Preferably, these charged oligosaccharides are linked to the backbone by an amide linkage which is formed by converting the reducing end of the oligosaccharide to a lactone and then selectively reacting the lactone with the functional amine groups on the homopolymer. Alternatively, the charged oligosaccharides are linked to the backbone by reductive amination, which involves a reaction between an amine group on the polymer and a terminal aldehyde on the oligosaccharide. The resulting linkage is a secondary amine.

The ratio of hydrophobic side chains to hydrophilic oligosaccharide chains on the polymer backbone is designed to achieve a hydrophilic to hydrophobic balance that allows the surfactant to adsorb onto the hydrophobic surface of the biomaterial and, preferably, that allows the hydrophilic side chains to extend from the surface of the biomaterial into a surrounding aqueous medium. The hydrophilic to hydrophobic balance depends on the density of the hydrophobic and hydrophilic side chains and the length of the hydrophobic side chains and hydrophilic side chains. Adhesion of the adsorbing polymer onto the hydrophobic surface of the biomaterial is enhanced by increasing the length, i.e., the number of methylene groups of the hydrophobic side chain, by increasing the density of the hydrophobic side chains relative to the hydrophilic side chains, and/or by reducing the length of the hydrophilic side chains. Thus, for a surfactant polymer in which the hydrophilic side chains are formed from dextran side chains composed of 9 glucose residues and from hydrophobic alkanoyl side chains comprising 4 methylene groups, the preferred ratio of hydrophilic side chains to a hydrophobic side chains is from about 2:1 to about 1:6, more preferably from about 1:1 to about 1:5 If the hydrophobic side chains comprise 10 methylene groups and the hydrophilic side chains are dextran side chains composed of 9 glucose residues, the preferred ratio of hydrophilic side chains to hydrophobic side chains from 3:1 to about 1:5, more preferably from about 2:1 to about 1:3.

In those cases where the ratio of oligosaccharide side chains to hydrophobic side chains on the resulting surfactant polymer is low, i.e., about 1:1, there may be some unreacted functional groups, such as for example residual amine groups, which are available to bind to plasma proteins. Preferably, such unreacted functional groups are blocked or capped by further reacting the resulting surfactant polymer with organic molecules that are small relative to the hydrophilic side chains prior to application of the surfactant to the biomaterial. Suitable small organic molecules are, by way of example, glucose, maltose, and acetaldehyde.

The surfactant polymer includes a plurality of hydrophilic oligopeptide side chains capable of interacting with specific protein receptors on the surface of animal cells such as, for example, endothelial cells. The oligopeptide side chains act as ligands for binding the cells to the surface of the biomaterial The oligopeptide side chains comprise from about 3 to about 30 amino acid residues. Preferably, the oligopeptide comprises the amino acid sequence RGD, more preferably RGDS, most preferably RGDSP. Alternatively, the oligopeptide comprises one of the following amino acid sequences: (i) RRAR, (ii) RRRKRR, (iii) PPRRARVT, or (iv) PPREVVPRPRP. In a preferred embodiment the oligopeptide comprises the sequence GSSSGRGDSPX, wherein X is alanine or another hydrophobic amino acid residue. The oligopeptide ligands are linked to the homopolymer backbone by an ester linkage, a secondary amine linkage, or an amide linkage.

Comblike surfactant polymers that comprise hydrophobic side chains, oligopeptide side chains, and oligosaccharide side chains are useful for providing a glycocalyx-like coating on the surface of a biomaterial. Such glycocalyx-like coatings prevent adhesion of plasma proteins to the coated surface of the biomaterial as compared to an uncoated hydrophobic surface of the biomaterial. Such coatings promote adhesion of selected cells, particularly endothelial cells, to the coated surface of the biomaterial.

The surfactants are used to coat one or more surfaces of a hydrophobic biomaterial, including a flexible, hydrophobic material. Such biomaterials are used to make implantable medical devices such as for example heart valves, stents, vascular grafts and catheters. Preferably, the surfactant is used to coat a surface which will come into contact with the blood or other body fluid of the patient following implantation of the device in the patient. The substrate is any material demonstrating biocompatibility and sufficient hydrophobicity to bind the surfactant, such as, for example, graphite and polyethylene. Other suitable biomaterials include, for example: polystyrene, polyesters, for example: Dacron®, carbon in pyrolytic carbon; polycarbonate; polymer membranes, for example, cellulose acetate, polyacrylonitrile; fluorocarbon polymers, for example Teflon®, Impra® and Gortex®; polysulfones; polyvinyl chloride; silicone rubber for example, Silastic®; silicone polymers; polypropylene; and polyurethanes. Suitable biomaterials also include nonpolymeric materials, such as for example, titanium, stainless steel, silicon, glass; and mixtures or composites thereof, that have been treated in a manner which renders their surfaces hydrophobic. The selection of the biomaterial depends upon the mechanical and functional properties required for forming an implantable biomedical device

The comblike surfactant polymers of the present invention, preferably, are soluble in water and are easily applied to the surface of the biomaterial. Application is achieved by immersing the biomaterial in a solution, preferably an aqueous solution, comprising the surfactant polymer . The surfactant spontaneously attaches to the hydrophobic surface of the polymeric biomaterial to provide a monolayer which alters the surface properties of the hydrophobic surface. The monolayer is bonded to the biomaterial via hydrophobic interactions and is able to withstand a shear stress of 75 dynes/cm² as determined using a rotating disc system followed by infrared spectroscopic surface analysis. Following adsorption of the surfactant to the hydrophobic surface, the surfactant may be air dried and stored in the dry state.

The following examples are for purposes of illustration only and are not intended to limit the scope of the claims which are appended hereto.

### COMPARATIVE EXAMPLEs

Surfactants comprising a poly(vinyl) backbone linked by amide linkages to a plurality of neutral oligosaccharide side chains and a plurality of hydrophobic side chains were prepared as follows:

### COMPARATIVE EXAMPLE 1:

### poly(N-vinyl dextran aldonamide-co-N-vinyl hexanoamide) (PNVDA-co-PNVH) (1:1.06)

### A. Materials and Methods

Acetaldehyde, formamide, formic acid, hexanoic acid, N-hydroxy succinimide and dicyclohexylcarbodiimide (DCCI) were purchased from Aldrich Chemical Co. and used as received. Dimethyl formamide (DMF) and dimethyl sulfoxide (DMSO) were purchased from Aldrich Chemical Co. and freshly distilled before use. before use. Strong anionic exchange resin Amberlite IR-400 (Sigma Chemical Co.) was rinsed with distilled water.

¹H-NMR spectra were recorded at ambient temperature, using a 200 MHz Varian Gemini-200 or a 400 MHz Bruker MSL-400 spectrometer, in parts per million downfield from tetramethylsilane (TMS) as internal standard and DMSO-d₆ or D₂O as solvent. Transmission IR spectra in the range of 400-0000 cm⁻¹ were recorded using a Digilab FTIR spectrometer. The materials were ground with KBr and pressed into pellets under reduced pressure. For each sample, 256 scans were collected with a resolution of 8 cm⁻¹. Gel permeation chromatography (GPC) was carried out using a HEMA-BIO100 GPC column equipped with a Rainin HPXL solvent delivery system and a DYNAMAX refractive index detector. The column was calibrated with dextrans with narrow molecular weight distribution. The flow rate was 1.0 mL/min. with distilled water as eluent.

### B. Synthesis of the Polymer

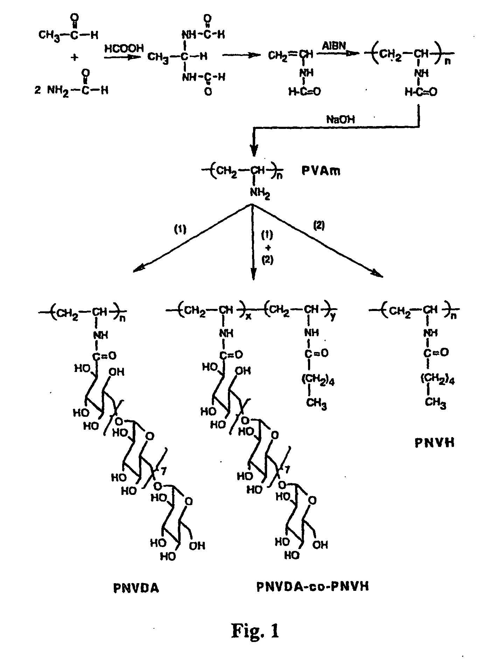

To obtain well-defined, defect-free, poly(vinyl amine) (PVAm), poly(N-vinyl formamide) was used as the precursor. As shown in the scheme of Figure 1, the synthesis of PVAm involved the following four steps: (i) synthesis of ethylidene bisformamide; (ii) pyrolysis of ethylidene bisformamide to N-vinyl formamide and formamide: (iii) polymerization of N-vinyl formamide to poly(N-vinyl formamide) and (iv) hydrolysis of poly(N-vinyl formamide) to PVAm, which is precipitated as a PVAm·HCl salt and then desalted by ion exchange chromatography. The synthetic route to poly(N-vinyl dextran aldonamide-co-N-vinyl hexanoamide) (PNVDA-co-PNVH) derivatives, is also shown in Figure 1. The product of each step was confirmed by spectroscopic analysis to ensure a well-defined final product of PVAm.

In the first step, acetaldehyde was condensed with formamide under acidic conditions to generate ethylidene bisformamide, which was purified by recrystallization. In the second step, ethylidene bisformamide was pyrolyzed to N-vinyl formamide and formamide. These two compounds are both liquids at ambient temperature, with very similar boiling points, but separation is not necessary. Thus, without any further purification, the N-vinyl formamide in the mixture was polymerized in isopropanol solution using AIBN as initiator. Poly(N-vinyl formamide) is water soluble with average molecular weight -10,000 as determined by GPC using dextrans as standards. In the last step, poly(N-vinyl formamide) was hydrolyzed to PVAm under basic conditions, and then precipitated in concentrated HCl solution as PVAm·HCl. The hydrolysis was carried out under basic, rather than acidic, conditions in order to achieve complete conversion. Under acidic conditions, positive charges build up along polymer chain during hydrolysis, which then limits further hydrolysis of the remaining formamide. Under basic conditions, no charges build up during hydrolysis, and complete conversion can be realized. No amide peaks were observed in the IR spectrum of PVAm·HCl, instead, very strong -NH₃⁺ peaks occur, indicating complete conversion of poly(N-vinyl formamide) to PVAm·HCl. The details of the synthesis were as follows:
i.) *Ethylidene Bisformamide*. To a solution of 45 g (1.0 mol) formamide and 50 g of formic acid heated to 40 °C with an oil bath, acetaldehyde (8.8 g, 0.2 mol) was added drop wise. The reaction mixture was stirred at 85°C for 4 h. Excess formamide and formic acid were evaporated by vacuum distillation with the bath temperature below 120°C. The yellow-brown residue was refluxed in acetone (300 mL) and then cooled to -70 °C to precipitate white crystals. The crystals were filtered and dried under vacuum at 70 °C to give 15.0 g (65%) of ethylidene bisformamide, which was further purified by recrystallization from a mixture of isopropanol and hexane. IR (KBr): 3211 cm⁻¹ (υ(N-H)), 2900 cm⁻¹, 3042 cm⁻¹ (υ(C-H) of CH₃ and CH), 1689 cm⁻¹ (amide I), 1545 cm⁻¹ (amide II). ¹H-NMR (DMSO-d₆, ppm): 1.24-1.31 (3H, -CH₃), 5.56-5.59 (1H, CH₃-CH-), 7.92-8.15 (2H, from two -CHO), 8.40-8.64 (2H, from two -CONH-).
ii.) *N-Vinyl Formamide*. A mixture of 6.96 g (60 mmol) ethylidene bisformamide and 2.0 g calcium carbonate was heated to 210°C under vacuum with a sodium nitrite bath. The pyrolyzed product was vacuum distilled to give 5.80 g (91%) of a slightly yellow liquid consisting of a 1:1 molar ratio of N-vinyl formamide and formamide. ¹H-NMR (DMSO-D₆, ppm): 4.19-4.76 (2H, CH₂=CH-), 6.60-6.94 (1H, CH₂=CH-), 7.23-7.47 (2H, -NH₂), 7.84-8.09 (2H, HCO-), 10.02 (1H, -CONH-).
iii.) *Poly(N-vinyl formamide)*. To 2.32 g of the 1:1 molar ratio mixture of N-vinyl formamide (20 mmol) and formamide (20 mmol), 5 mL isopropanol was added. The reaction mixture was purged with argon and freeze-thawed 3 times using liquid nitrogen to eliminate oxygen. 2, 2'-Azobisisobutyronitrile (AIBN, 32 mg, 0.12 mmol) was added to the reaction solution, which was then refluxed for 4 h under argon. Isopropanol was removed by rotary vacuum evaporation. The residue was dissolved in a small amount of water and precipitated in acetone. The precipitate was filtered and dried under vacuum at 70 °C overnight to give 1.3 g (89%) of poly(N-vinyl formamide). GPC measurement showed that the number average molecular weight was -10,000. IR (KBr): 1677 cm⁻¹ (amide I), 1538 cm⁻¹ (amide II). ¹H-NMR (D₂O, ppm): 1.57 (2H, -CH₂-), 3.79 (1H, -CH-), 7.90 (1H, HCO-).
iv) *Poly(vinyl amine)·hydrochloride (PVAm·HCl)*. To a 4 mL aqueous solution of 0.85 g (12 mmol) of poly(N-vinyl formamide), 4 mL aqueous NaOH (0.72 g, 18 mmol) solution was added. The mixture was stirred at 80°C for 6 h under nitrogen. After cooling to ambient temperature, the solution was acidified with concentrated HCl, to precipitate the PVAm·HCl salt. The precipitate was washed with methanol to neutral pH, and dried under vacuum to give 0.62 g (65%) PVAm·HCl. IR (KBr) -3420 cm⁻¹ (υ(N-H) of NH₂), ∼3000 cm⁻¹ (wide and strong overlapping peak from υ(N-H) of -NH₃⁺ and υ(C-H) of CH₂ and CH), 1606 and 1512 cm⁻¹ ((δₐₛ(N-H) and δₛ(N-H) of -NH₃⁺). No amide peaks were observed. ¹H-NMR (D₂O, ppm): 2.16 (2H, -CH₂-), 3.74 (1H, -CH-).

*Poly(vinyl amine) (PVAm)*. PVAm was obtained by passing the aqueous solution of PVAm·HCl through a strong anionic exchange column (Amberlite IR-400), followed by lyophilizing the eluate. IR (KBr): ∼3300 cm⁻¹ (u(N-H) of -NH₂), 2870 and 2930 cm⁻¹ (u(C-H) of CH₂ and CH), 1591 cm⁻¹ (δ(N-H) of -NH₂). ¹H-NMR (D₂O, ppm): 1.22 (2H, -CH₂-), 2.85 (1H, -CH-). ¹H-NMR (DMSO-D₆, ppm): 1.18 (-CH₂-), 2.92(-CH-), 2.6 (-NH₂) (small amount of PVAm can be dissolved in DMSO-D₆ at 60°C), ¹H-NMR (DMSO-D₆ with a drop of D₂O, ppm): 1.20 (2H, -CH₂-), 2.88(1H, -CH-).

The PVAm is readily soluble in water and methanol. From the ¹H-NMR of PVAm in D₂O, the peak integration of -CH₂-, -CH- agree with the predicted molecular structure. The proton resonance peak of -NH₂ was not observed for PVAm in D₂O, because of rapid proton exchange between -NH₂ and any H₂O in D₂O. The proton resonance peak of -NH₂ (2.6 ppm) was observed when using vigorously dried DMSO-D₆ as solvent. However, after adding a drop of D₂O into the PVAm-DMSO-D₆ solution, the peak disappears. The proton peaks of -CH₂- and -CH- are broad because of the poor solubility of PVAm in DMSO. In addition, the proton peak of the trace H₂O is shifted, probably due to the interaction between H₂O and DMSO.

### C. Synthesis of N-Hexanoyloxy Succinimide.

To a solution of 4.64 g (0.04 mol) of hexanoic acid and 5.75 g (0.05 mol) of N-hydroxy succinimide in 100 mL distilled DMF, 10.3 g (0.05 mol) of DCCl was added. The mixture was cooled with an ice-water bath and stirred for 5 h. The dicyclohexyl urea (DCU) precipitate was removed by filtration, and the DMF solvent was removed by vacuum rotary evaporation. The yellow oil residue was washed with water and hexane to yield a white solid, which was vacuum dried at 78°C to give 6.34 g (74%) of N-hexanoyloxy succinimide. IR (KBr): 1816 cm⁻¹ (u(C=O) of ester), 1745-1786 cm⁻¹ (υ(C=O) of imide). ¹H-NMR (DMSO-d⁶, ppm): 0.86 (3 H, - CH₃)_{,} 1.31 (4H, -(CH₂)₂CH₃), 1.62 (2H, -CH₂CH₂COO-), 2.64 (2H, -CH₂COO-), 2.80 (4H, - COCH₂CH₂CO-).

### D. Synthesis of Dextran Lactone

Dextran lactone (M_{w} = 1600, M_{w}/Mₙ = 1.16, DP = 9) was prepared as described in Zhang, T.; Marchant, R. E. Macromolecules 1994, 27(25), 7302-7308, which is specifically incorporated herein by reference.

### E. Synthesis of Surfactant polymer

(PNVDA-co-PNVH) (1:1.06) was prepared by reacting amino groups of PVAm with dextran lactone and N-hexanoyloxy succinimide simultaneously at a molar feed ratio of dextran lactone to N-hexanoyloxy succinimide of 1:1. Specifically, 0.54 g (0.4 mmol) of dextran lactone in 6 mL DMSO was added to a 2 mL methanol solution containing 34.2 mg of PVAm (0.8 mmol amino groups) and 85.2 mg (0.4 mmol) of N-hexanoyl succinimide. After stirring for 4 h at room temperature, the solution was heated to 70 °C with an oil bath and stirred for 2 days. The product solution was concentrated by vacuum distillation and precipitated by the addition of acetone. The precipitate was filtered and dried under vacuum at 78 °C overnight to give 0.53 g (81% yield) raw product. This was purified by extensive dialysis against water as described previously.

### COMPARATIVE EXAMPLE 2

Poly(N-vinyl dextran aldonamide-co-N-vinyl hexanoamide) (PNVDA-co-PNVH) (1:3.7) (PNVDA-co-PNVH) (1:3.7)was prepared as described above in comparative example 1, except the molar feed ratio of dextran lactone to N-hexanoyloxy succinimide was 1:2

### COMPARATIVE EXAMPLE 3

### Poly(N-vinyl dextran aldonamide-co-N-vinyl hexanoamide) (PNVDA-co-PNVH) (1:5)

(PNVDA-co-PNVH) (1:5)was prepared as described above in comparative example 1, except the molar feed ratio of dextran lactone to N-hexanoyloxy succinimide was 1:4.

### COMPARATIVE EXAMPLE 4

### Poly(N-vinyl dextran aldonamide-co-N-vinyl dodecanoamide) (PNVDA-co-PNVL) (1:1.5)

(PNVDA-co-PNVL) (1:1.5) was prepared as described above in comparative example 1 except that PVAm was reacted simultaneously with dextran lactone and N-lauroyloxy succinimide. The molar feed ratio of dextran lactone to N-lauroyloxy succinimide was 1:2. -

### COMPARATIVE EXAMPLE 5

### Poly(N-vinyl dextran aldonamide-co-N-vinyl dodecanoamide) (PNVDA-co-PNVL) (1:1.06)

(PNVDA-co-PNVL) (1:1.06) was prepared as described above in comparative example 1 except that PVAm was reacted simultaneously with dextran lactone and N-lauroyl succinimide. The molar feed ratio of dextran lactone to N-lauroyl succinimide was 1:1.

### Characterization of the Surfactant Polymers of Comparative examples 1-5

### A. FITR and ¹H-NMR spectroscopies

The surfactant polymers of comparative examples 1-3 were characterized by Fourier transform infrared (FTIR) and ¹H-NMR spectroscopies and elemental analysis to confirm purity and structure. The results were as follows:
IR: 3310 cm⁻¹ (υ(O-H)), 2930-2874 cm⁻¹ (υ(C-H) of CH₂ and CH), 1643 cm⁻¹ (amide 1), 1547 cm⁻¹ (amide II), 1149-1032 cm⁻¹ (υ(C-O)). ¹H-NMR (DMSO-D₆, ppm): 0.85 (-CH₃ of hexanoyl groups, CH₃(CH₂)₃CH₂CO-), 1.1-1.6 ((CH₂)₃ of CH₃(CH₂)₃CH₂CO- and -CH₂- from PVAm backbone), 2.1 ( CH₂ of CH₃(CH₂)₃CH₂CO-), 3.0-4.1 (-CH- of PVAm backbone and all dextran CH's and CH₂'s except the ones at the glycosidic linkages), 4.1-5.3 (all dextran OH's and CH at the glycosidic linkages), 7-8 (-NH- of amide linkages).

The IR results demonstrated qualitatively the expected comb-like structure of PNVDA-co-PNVH. The ¹H-NMR spectra of the purified surfactants show proton peaks derived from PVAm, dextran and hexanoyl groups, confirming the expected composition for the surfactants.

The compositions for PNVDA-co-PNVHs prepared from the 3 different molar feed ratios were estimated, based on proton integration of ¹H-NMR spectra. The measured compositions are, as expected, lower than the extreme theoretical values calculated by assuming syndiotactic The polymer surfactants with varied hydrophilic/hydrophobic balances were designated as PNVDA-co-PNVH (1:1), PNVDA-co-PNVH (1:3.7), and PNVDA-co-PNVH (1:5). PNVDA-co-PNVH (1:1) and PNVDA-co-PNVH (1:3.7) are soluble in water, DMF and DMSO, while PNVDA-co-PNVH (1:5) is not readily soluble in water, but is soluble in DMSO. However, PNVDA-co-PNVH (1:5) becomes partially soluble in water with vigorous sonication ( 1.5 h). An opaque solution can be prepared at low concentration (∼1 mg/ml).

Surfactants comprising a poly(vinyl) backbone linked by secondary amine linkages to a plurality of peptide side chains and a plurality of hydrophobic side chains were prepared by simultaneously reacting PVAm with different molar ratios of alkyl aldehydes and with peptides linked to a spacer molecule having a reactive aldehyde at the free end thereof. A suitable spacer molecule is a glutaric dialdehyde molecule that reacts with the free amine end of the peptide and the amine group on the polymer. Another suitable spacer molecules include poly(ethylene oxide) (PEO) dialdehyde spacer or other similar bifunctional molecule. The peptide itself is synthesized to either have a free carboxyl end group or an end that is capped.

Synthesis of the poly(vinyl amine) (PVAm) polymer surfactant requires two steps: (1) modifying the peptide to create a terminal aldehyde group; and (2) simultaneously coupling the modified peptide and an alkyl aldehyde to the polymer backbone using a standard Schiff base reaction with reduction to a secondary amine. In a preferred embodiment, the surfactant polymer comprises two different types of hydrophilic side chains, one being a peptide and the other being an oligosaccharide. For such surfactant polymers, the molar amount of peptide and saccharide can be varied, but the total molar amount of hydrophilic groups should result in a 1:0.5 to 1:2 ratio with hexanal, for solubility reasons.

### EXAMPLE 1

### Poly(N-vinyl-5-peptidyl-pentylamine-co-N-vinyl hexyl amine (PVAm(Pep:Hex) (1:1.75)

### A. Materials

All solvents (synthesis grade N,N-dimethyl formamide (DMF), dichloromethane, 20% piperidine/DMF, and DIPEA), resins, and activator were purchased from Perseptive Biosystems. The FMOC protected amino acids were obtained from AnaSpec. The scavengers (phenol, 1,2 ethanedithiol, triisopropylsilane), sodium cyanoborohydride (NaCNBH₃) and hexanal (Aldrich) were used as received. Ultrapure water was delivered from a Millipore-RO system, with hydrocarbon content < 5ppb. Poly(vinyl amine) was prepared as described in comparative example 1. All other reagents were used as received unless otherwise stipulated.

### B. Synthesis of the Peptide

The initial peptide was synthesized with a solid phase peptide synthesizer (SPPS), utilizing common solvents, packing resins and capped amino acids. This peptide is an eleven amino acid molecule having the following sequence: 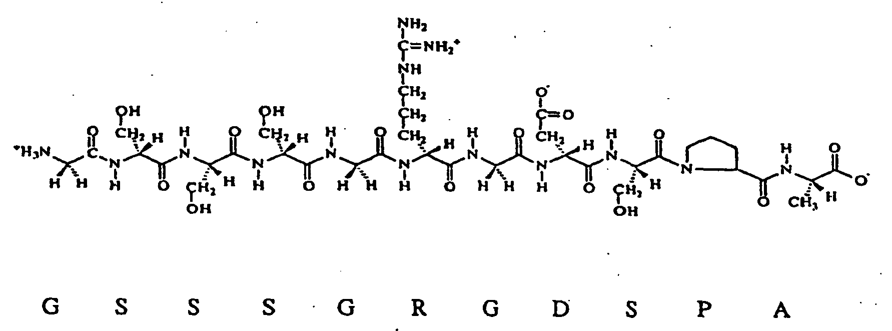 The peptide was then purified by preparatory scale-high performance liquid chromatography (HPLC and
characterized for composition by mass spectroscopy and HPLC. An average yield per batch is about 60-80 mg of pure (>98%) product. The product was then stored in a -20°C freezer to minimize moisture uptake.

### C. Peptide Modification

In a small bottom flask, 25.6 mg (0.256 mmol) glutaraldehyde and 3.2 mg (0.0512 mmol) NaCNBH₃ was dissolved in 3 mL ultrapure water. A solution of 50 mg (0.0512 mmol) pure peptide in 1 mL ultrapure water was added dropwise. The pH of the solution was adjusted to 6 using HCl, and the flask was sealed and allowed to stir at room temperature for 4 hours. The peptide was purified from excess glutaraldehyde by extensive dialysis against pure water using Spectra Pro 3 regenerated cellulose membrane with 500 molecular weight cutoff.

### D. Surfactant Polymer Synthesis.

To prepare PVAm(PEP:HEX)(1:1.75), 25.4 mg (0.024 mmol) modified peptide was dissolved in 0.20 mL ultrapure water. (Water must be less than 10% of the final solution volume for the sample to precipitate out in acetone).To this mixture, 3.6 mg (0.036 mmol) hexanal and 3.75 mg (0.06 mmol) NaCNBH₃ in 2.0 mL ethanol was added. Finally, 2.565 mg (0.06 mmol) PVAm in 2 mL ethanol was added to the reaction flask dropwise. The pH of the solution was adjusted to 6 using HCl, and the solution stirred for 24 hours at room temperature. The solution was precipitated in 100 mL acetone and dried at room temperature under vacuum overnight. The raw product was further purified by dialyzing against ultrapure water using Spectra Pro 3 regenerated cellulose membranes with 3500 molecular weight cutoff. The purified sample was then lyophilized to obtain the final surfactant product. In general, peptide surfactant polymers with peptide to hexanal ratios between (1:0.5) and (1:2) would be the desirable range.

### EXAMPLE 2

### Poly(N-vinyl-5-peptidyl-pentylamine-co-N-vinyldextranaldonamine-co-N-vinyl hexyl amine (PVAm(Pep:Dex:Hex))

A surfactant polymer comprising a plurality of hydrophobic side chains, a plurality of oligosaccharide side chains, and a plurality of peptide chains were prepared by reacting the modified peptide and, dextran lactone, and hexanal simultaneously with the PVAm as described above in example 1. The molar ratios of the peptide, dextran lactone, and hexanal were 1:1:3

### EXAMPLE 3

### Poly(N-vinyl-5-peptidyl-pentylamine-co-N-vinyldextranaldonamine-co-N-vinyl hexyl amine (PVAm(Pep:Dex:Hex))

A surfactant polymer comprising hydrophobic side chains, peptide side chains, and oligosaccharide side chains was prepared by reacting the modified peptide, maltolactone, and hexanal simultaneously with the PVAm as described above in example 1.
The molar feed ratios of the peptide, dextran lactone, and hexanal were 3:1:6. Characterization of the Surfactant Polymers of Examples 1-3

### A. FITR and ¹H-NMR spectroscopies

The purity of the peptide surfactant polymers after dialysis was verified using IR spectroscopy, which showed that no residual aldehyde peak (at 1740 cm-) was present in the spectrum. The final peptide-to-hexanal ratio was determined using 'H-NMR spectroscopy. This was done by taking the integrals of the proton peaks that correspond to each ligand and calculating the final value.

### B Alteration of Surface Properties of Materials Coated with the Surfactant Polymers of Examples 1-3

The surfactant polymers were shown to be surface active on hydrophobic materials, such as octadecyltrichlorosilane (OTS)-derivatized glass. The surfactant was first dissolved in a water/acetonitrile mixture, then the OTS glass was submerged in the solution for 24 hour adsorption. The sample was removed, dried overnight and water contact angle measurements were taken. Typically, the water contact angle dropped from 110 degrees to 20-30 degrees, indicating the successful adsorption of surfactant to the surface. The surfactant-coated OTS was then placed in a low flow chamber where water is constantly flowing over the sample for 24 hours. This procedure tests the adhesion stability of the surfactant on the hydrophobic OTS surface.. After drying the sample, contact angle measurements are again taken. Typically the water contact angle increase only about 10 degrees, to about 30-40 degrees, indicating that the surfactant remains firmly attached to the hydrophobic surface.

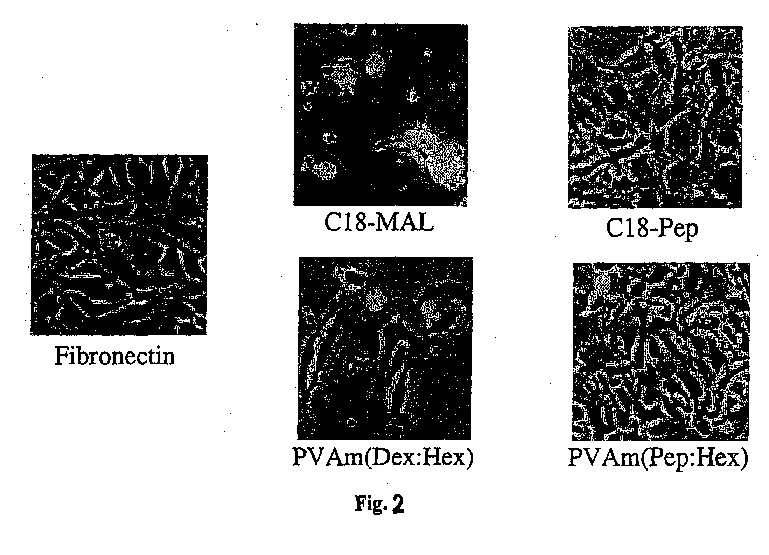

The surfaces with adsorbed peptide surfactant polymers were shown to promote endothelial cell growth and proliferation. Human umbilical vein endothelial cells (HUVEQ) in HEPES buffer were added to containers having OTS samples coated with the surfactant polymers of examples 1-3. Phase contrast and fluorescent microscopic images were taken after 3, 24 and 72 hours of incubation time. The results at 72 hours for the phase contrast images are shown in Figure 2. Note that fibronectin coated OTS serves as a positive control for HUVEC growth and proliferation. No cell growth was observed on surfaces coated with the surfactant polymer of comparative example 2 or maltose linear surfactants, as shown by phase contrast microscopy. This result serves as a demonstration that the surfactant polymers prepared as comparative examples, with a poly(vinyl) backbone linked by amide linkages to a plurality of neutral oligosaccharide side chains and a plurality of hydrophobic side chains, prevent adhesion of proteins and cells. However, for the peptide surfactant coated surfaces, cell growth was similar to that on fibronectin. Cell growth was also quantified using fluorescent staining for the actin fibers and focal adhesion points to the surface. As shown in Figure 2, the peptide surfactant polymers grew healthy cells with long actin stress fibers and many focal adhesion points. As the peptide-to-dextran ratio decreased, the cell viability decreased as well. These results indicate that the peptide surfactant polymers have a well-defined structure, stably adsorb to hydrophobic surfaces, and promote cell growth and proliferation.

## Claims

1. A comblike surfactant polymer comprising:
a) a polymeric backbone of repeating monomeric units;
b) a plurality of hydrophobic side chains comprising from 2 to 18 methylene groups, said plurality of hydrophobic side chains being linked to said polymeric backbone by ester linkages, secondary amine linkages, amide linkages, or combinations thereof; and
c) a plurality of hydrophilic side chains linked to said polymeric backbone by ester linkages, secondary amine linkages, amide linkages, or combinations thereof, said hydrophilic side chains comprising an oligopeptide of from 3 to 30 amino acid residues, said oligopeptide having an amino acid sequence which interacts with protein receptors on the surface of cells.

2. The surfactant polymer of claim 1 wherein said polymeric backbone is a derivatized poly (vinyl amine), or a derivatized poly (vinyl alcohol) or a derivatized polylysine.

3. The surfactant polymer of claim 1 wherein said polymeric backbone is linked to said plurality of hydrophobic side chain by amide linkages;
and wherein said polymeric backbone is linked to said plurality of hydrophilic side chains by amide linkages.

4. The surfactant polymer of claim 1 wherein said polymeric backbone is linked to said plurality of hydrophobic side chains by secondary amine linkages; and
wherein said polymeric backbone is linked to a plurality of said oligopeptide side chains by secondary amine linkages.

5. The surfactant polymer of claim 1 further comprising a second plurality of hydrophilic side chains selected from neutral oligosaccharide side chains having a weight average molecular weight of less than 7,000, charged oligosaccharide chains having a weight-average molecular weight of less than 10,000, and combinations thereof.

6. The surfactant polymer of claim 1 wherein said oligopeptide side chains comprise an amino acid sequence selected from the group consisting of: RGD, RRAR, RRRKRR, PPRRARVT, and PPREVVPRPRP.

7. The surfactant polymer of claim 1 wherein the ratio of hydrophilic side chains to hydrophobic side chains is from 3:1 to 1:6.

8. A method of promoting adhesion of animal cells to a surface of a hydrophobic bio-material comprising providing a polymer according to any of claims 1 to 7 and attaching it to the surface of said material.

9. A method according to claim 8 which is for rendering said hydrophobic material more susceptible to the attachment of endothelial cells, wherein in said comblike surfactant polymer,
(a) said plurality of hydrophobic side chains are linked to said polymeric backbone by secondary amine linkages, amide linkages; or combinations thereof and
(b) said plurality of hydrophilic side chains are linked to said polymeric backbone by secondary amine linkages, and said oligopeptide has an amino acid sequence which interacts with protein receptors on the surface of endothelial cells.

## Patentansprüche

1. Kammähnliches Tensidpolymer, umfassend:
a) eine Polymerhauptkette aus Monomer-Grundeinheiten;
b) eine Vielzahl hydrophober Seitenketten, die 2 bis 18 Methylengruppen umfassen, wobei die Vielzahl hydrophober Seitenketten über Esterbindungen, sekundäre Aminbindungen, Amidbindungen oder Kombinationen davon an die Polymerhauptkette gebunden ist; und
c) eine Vielzahl hydrophiler Seitenketten, die über Esterbindungen, sekundäre Aminbindungen, Amidbindungen oder Kombinationen davon an die Polymerhauptkette gebunden sind, wobei die hydrophilen Seitenketten ein Oligopeptid aus 3 bis 30 Aminosäureresten umfassen, wobei das Oligopeptid eine Aminosäuresequenz aufweist, die mit Proteinrezeptoren auf der Oberfläche von Zellen wechselwirkt.

2. Tensidpolymer nach Anspruch 1, worin die Polymerhauptkette ein derivatisiertes Poly(vinylamin) oder ein derivatisierter Poly(vinylalkohol) oder ein derivatisiertes Polylysin ist.

3. Tensidpolymer nach Anspruch 1, worin die Polymerhauptkette über Amidbindungen mit der Vielzahl hydrophober Seitenketten verbunden ist und worin die Polymerhauptkette über Amidbindungen mit der Vielzahl hydrophiler Seitenketten verbunden ist.

4. Tensidpolymer nach Anspruch 1, worin die Polymerhauptkette über sekundäre Aminbindungen mit der Vielzahl hydrophober Seitenketten verbunden ist und worin die Polymerhauptkette über sekundäre Aminbindungen mit der Vielzahl hydrophiler Seitenketten verbunden ist.

5. Tensidpolymer nach Anspruch 1, weiters umfassend eine zweite Vielzahl hydrophiler Seitenketten, die aus neutralen Oligosaccharidseitenketten mit einem gewichtsmittleren Molekulargewicht von weniger als 7.000, geladenen Oligosaccharidseitenketten mit einem gewichtsmittleren Molekulargewicht von weniger als 10.000 und Kombinationen davon ausgewählt sind.

6. Tensidpolymer nach Anspruch 1, worin die Oligopeptidseitenketten eine aus der aus RGD, RRAR, RRRKRR, PPRRARVT und PPREWPRPRP bestehenden Gruppe ausgewählte Aminosäuresequenz umfassen.

7. Tensidpolymer nach Anspruch 1, worin das Verhältnis zwischen hydrophilen Seitenketten und hydrophoben Seitenketten 3:1 bis 1:6 beträgt.

8. Verfahren zur Förderung der Haftung von tierischen Zellen an einer Oberfläche eines hydrophoben Biomaterials, umfassend die Bereitstellung eines Polymers nach einem der Ansprüche 1 bis 7 und das Anbringen desselben an der Oberfläche des Materials.

9. Verfahren nach Anspruch 8, das dazu dient, das hydrophobe Material für die Anhaftung von Endothelzellen besser geeignet zu machen, worin in dem kammähnlichen Tensidpolymer
(a) die Vielzahl hydrophober Seitenketten über sekundäre Aminbindungen, Amidbindungen oder Kombinationen davon an die Polymerhauptkette gebunden ist und
(b) die Vielzahl hydrophiler Seitenketten über sekundäre Aminbindungen an die Polymerhauptkette gebunden ist und das Oligopeptid eine Aminosäuresequenz aufweist, die mit Proteinrezeptoren auf der Oberfläche von Endothelzellen wechselwirkt.

## Revendications

1. Polymère d'agent de surface semblable à un peigne comprenant:
a) une épine dorsale polymère d'unités monomères de répétition;
b) une pluralité de chaînes latérales hydrophobes comprenant de 2 à 18 groupes de méthylène, ladite pluralité de chaînes latérales hydrophobes étant liée à ladite épine dorsale polymère par des liaisons ester, des liaisons d'amines secondaires, des liaisons d'amide, ou leurs combinaisons; et
c) une pluralité de chaînes latérales hydrophiles liées à ladite épine dorsale polymère par des liaisons ester, liaisons d'amines secondaires, liaisons d'amide ou leurs combinaisons, lesdites chaînes latérales hydrophiles comprenant un oligopeptide de 3 à 30 résidus d'acides aminés, ledit oligopeptide ayant une séquence d'acides aminés qui est en interaction avec des récepteurs de protéine sur la surface de cellules.

2. Polymère d'agent de surface selon la revendication 1, où ladite épine dorsale polymère est une poly(vinyl amine) dérivé, ou un poly (vinyl alcool) dérivé ou une polylysine dérivée.

3. Polymère d'agent de surface selon la revendication 1, où ladite épine dorsale polymère est liée à ladite pluralité de chaînes latérales hydrophobes par des liaisons d'amide;
et où ladite épine dorsale polymère est liée à ladite pluralité de chaînes latérales hydrophiles par des liaisons d'amide.

4. Polymère d'agent de surface selon la revendication 1, où ladite épine dorsale polymère est liée à ladite pluralité de chaînes latérales hydrophobes par des liaisons d'amines secondaires; et
où ladite épine dorsale polymère est liée à une pluralité desdites chaînes latérales d'oligopeptide par des liaisons d'amine secondaires.

5. Polymère d'agent de surface selon la revendication 1, comprenant en outre une deuxième pluralité de chaînes latérales hydrophiles sélectionnées parmi des chaînes latérales d'oligosaccharides neutres ayant une masse moléculaire moyenne en poids inférieure à 7000, de chaînes d'oligosaccharides chargées ayant une masse moléculaire moyenne en poids inférieure à 10 000 et leurs combinaisons.

6. Polymère d'agent de surface selon la revendication 1, où lesdites chaînes latérales d'oligopeptide comprennent une séquence d'acides aminés sélectionnée dans le groupe consistant en: RGD, RRAR; RRRKRR, PPRRARVT et PPREVVPRPRP.

7. Polymère d'agent de surface selon la revendication 1, où le rapport des chaînes latérales hydrophiles aux chaînes latérales hydrophobes est de 3:1 à 1:6.

8. Méthode pour encourager l'adhésion de cellules animales à une surface d'un bio-matériau hydrophobe comprenant la réalisation d'un polymère selon l'une quelconque des revendications 1 à 7 et la fixation de celui-ci à la surface dudit matériau.

9. Méthode selon la revendication 8, destinée à rendre ledit matériau hydrophobe plus susceptible à l'attachement de cellules endothéliales, où dans ledit polymère d'agent de surface semblable à un peigne,
(a) ladite pluralité de chaînes latérales hydrophobes sont liées à ladite épine dorsale polymère par des liaisons d'amine secondaires, des liaisons d'amide ou leurs combinaisons et
(b) ladite pluralité de chaînes latérales hydrophiles sont liées à ladite épine dorsale polymère par des liaisons d'amine secondaires, et ledit oligopeptide présente une séquence d'acides aminés qui est en interraction avec des récepteurs de protéine sur la surface de cellules endothéliales.
